# EUROPEAN PATENT APPLICATION

(11) **EP 4 715 827 A1**
(43) Date of publication of application: **25.03.2026**
(21) Application number: 24807172.2
(22) Date of filing: 11.05.2024
(51) Int. Cl.: G16B 30/00

(54) **SEQUENCE CLUSTERING METHOD**

(30) Priority: 14.05.2023 JP 2023079746
(71) Applicant: Nihon Biodata Corporation, Kawasaki-shi, Kanagawa 213-0012 (JP)
(72) Inventor: OGATA Norichika, Kawasaki-shi, Kanagawa 213-0012 (JP)
(74) Representative: van Trier, Norbertus Henricus Gerardus
(86) International application number: PCT/JP2024/017548
(87) International publication number: WO 2024/237220

(57) **Abstract**

[Problem to be Solved] To provide a sequence clustering method that can easily cluster sequences of sequences included in a database in relation to specimen-derived reads.

[Solution] A sequence clustering method comprising: a read acquisition step of acquiring reads derived from specimens; a read ID acquisition step of acquiring identification information on the reads; an ID group acquisition step of checking reads against references included in a database and acquiring ID groups; a high-frequency read ID acquisition step of obtaining the number of pieces of read identification information included in ID groups and obtaining high-frequency read identification information; a high-frequency read identification information acquisition step of obtaining high-frequency reference identification information; a relevant reference information acquisition step of acquiring, using high-frequency reference identification information, information including organism species of high-frequency reference sequences stored in association with the high-frequency reference identification information from the database; a reference cluster step of allowing high-frequency reference identification information to be replaced with first cluster identification information; and a replaced ID group acquisition step of converting high-frequency reference identification information included in ID groups into first cluster identification information.

## Description

### TECHNICAL FIELD

This invention relates to a sequence clustering method and the like.

### BACKGROUND ART

Patent Literature JP 2022-176173 A describes a method for classifying biological sequences.

For example, in the field of manufacturing pharmaceuticals using biological materials such as cells, so-called bio-pharmaceuticals, for the purpose of managing the safety of the pharmaceuticals to be manufactured, it is sometimes examined whether the final product is contaminated with viruses or components derived from manufacturing host cells. Sequencing is performed on specimens derived from intermediate products and final products of bio-pharmaceutical manufacturing, and searches are sometimes conducted against databases containing base sequences obtained from specimens that previously contained viruses. As a result of the aforementioned search, when base sequences derived from any specimen contain virus-derived base sequences, the risk of virus contamination in any specimen can be recognized. Therefore, to implement virus safety management of intermediate products and final products of bio-pharmaceutical manufacturing, databases containing organism species names and base sequences obtained from specimens containing those organism species can be utilized.

When attempting to create a database by collecting base sequences obtained from specimens containing viruses, it is necessary to design the amount of base sequences included in the database. This is because the base sequences of viruses that have been sequenced to date are enormous, and there are many duplications in base sequences derived from specimens obtained at different times and places for the same type of virus. While databases containing many of the aforementioned duplications have high comprehensiveness and low risk of failing to find virus-derived sequences that are actually contained in search results, they have the disadvantage that when base sequence searches are performed using the database, one read obtains matches to the same type of virus base sequences multiple times, making aggregation difficult.

### CITATION LIST

### PATENT LITERATURE

Patent Literature 1 JP 2022-176173 A

### SUMMARY OF INVENTION

### TECHNICAL PROBLEM

This invention aims to provide a sequence clustering method capable of easily clustering nucleotide sequences included in a database in relation to specimen-derived reads.

### SOLUTION TO PROBLEM

This invention is based on the finding that clustering of references can be performed by measuring similarity (determining relationships) between references using reads.

The sequence clustering method includes a read acquisition step (S110), a read ID acquisition step (S120), an ID group acquisition step (S130), a high-frequency read ID acquisition step (S140), a high-frequency read identification information acquisition step (S150), a relevant reference information acquisition step (S160), a reference cluster step (S170), and a replaced ID group acquisition step (S180).

The read acquisition step (S110) is a step of acquiring reads that are base sequences derived from specimens. An example of a specimen is a bio-pharmaceutical.

The read ID acquisition step (S120) is a step of acquiring identification information on the reads.

The ID group acquisition step (S130) checks, with reference to a database including a plurality of base sequences of references and storing the base sequence of each reference in association with an organism species and identification information on each reference, the reads against the references included in the database, and acquires an ID group that is a group of the read identification information and the reference identification information in a case where the base sequences of the references include the base sequences of the reads. Examples of base sequences of references included in the database are base sequences derived from viruses, molds, and bacteria that may be included in bio-pharmaceuticals.

The high-frequency read ID acquisition step (S140) is a step of obtaining the number of pieces of read identification information included in ID groups and obtaining high-frequency read identification information that is identification information on high-frequency reads that are reads with high appearance rates.

The high-frequency read identification information acquisition step (S150) is a step of obtaining high-frequency reference identification information that is the reference identification information grouped with the high-frequency read identification information included in the ID groups.

The relevant reference information acquisition step (S160) is a step of acquiring, using the high-frequency reference identification information, information including an organism species of the high-frequency reference sequences stored in association with the high-frequency reference identification information from the database.

The reference cluster step (S170) is a step of allowing the high-frequency reference identification information to be replaced with first cluster identification information that is a new piece of identification information.

The replaced ID group acquisition step (S180) is a step of acquiring a replaced ID group by converting the high-frequency reference identification information included in the ID groups into the first cluster identification information.

A preferred example of the ID group acquisition step (S130) includes a first read group acquisition step (S131) and a first ID group acquisition step (S132).
The first read group acquisition step (S131) is a step of checking the reads against a first host-derived reference that is a reference derived from a host of the specimen, and obtaining a first read group of reads other than reads whose base sequences are included in the first host-derived reference sequence.

The first ID group acquisition step (S132) is a step of checking the first read group against the references included in the database, and acquiring a first ID group. This example preferably further includes a second read group acquisition step (S133) and a second ID group acquisition step (S134).

The second read group acquisition step (S133) is a step of checking the reads against a second host-derived reference that is a reference derived from the host of the specimen, and obtaining a second read group of reads other than reads whose base sequences are included in the second host-derived reference sequence. The second host-derived reference sequence and the first host-derived reference sequence are different sequences.

The second ID group acquisition step (S134) is a step of checking the second read group against the references included in the database, and acquiring a second ID group.

A preferred example of the ID group acquisition step (S130) further includes a deduplication step (S135). The deduplication step (S135) is a step of leaving, in a case where there are a plurality of ID groups with an identical pair of the read identification information and the reference identification information, only one of the ID groups and removing the other ID groups.

A preferred example of the sequence clustering method may perform an iteration step (S190) that performs the high-frequency read ID acquisition step, high-frequency read identification information acquisition step, relevant reference information acquisition step, and replaced ID group acquisition step using the replaced ID group in a case where the replaced ID group includes a plurality of pieces of identification information on a certain read.

A computer for performing sequence clustering comprises a processor and a memory coupled to the processor. The memory stores a program including a command. When executed by the processor, the command causes the computer to execute a sequence clustering method including the read acquisition step (S110), read ID acquisition step (S120), ID group acquisition step (S130), high-frequency read ID acquisition step (S140), high-frequency read identification information acquisition step (S150), relevant reference information acquisition step (S160), reference cluster step (S170), and replaced ID group acquisition step (S180). When executed by the processor, the command may further cause the computer to execute the iteration step (S190).

The above program is a program for causing a computer to execute a sequence clustering method including the read acquisition step (S110), read ID acquisition step (S120), ID group acquisition step (S130), high-frequency read ID acquisition step (S140), high-frequency read identification information acquisition step (S150), relevant reference information acquisition step (S160), reference cluster step (S170), and replaced ID group acquisition step (S180). The program may further cause the computer to execute the iteration step (S190).

A non-transitory information recording medium stores the above program.

### ADVANTAGEOUS EFFECTS OF INVENTION

According to this invention, sequences of sequences included in a database can be easily clustered in relation to specimen-derived reads. Therefore, for example, whether a specimen contains viruses can be easily analyzed.

### BRIEF DESCRIPTION OF DRAWINGS

[Fig. 1] Fig. 1 is a flowchart showing an example of steps of a clustering method.
[Fig. 2A] Fig. 2 shows an example for explaining the clustering method of this invention. Fig. 2A shows examples of read and reference sequences and identification information.
[Fig. 2B] Fig. 2B is a diagram for explaining an ID group acquisition step including a first read group acquisition step and a first ID group acquisition step.
[Fig. 2C] Fig. 2C is a diagram for explaining steps after acquiring ID groups.
[Fig. 2D] Fig. 2D is a diagram for explaining an example of clustering processing after obtaining a conversion table.
[Fig. 2E] Fig. 2E is a diagram for explaining an example of steps up to obtaining a second conversion table in the iteration step.
[Fig. 2F] Fig. 2F is a diagram for explaining an example of steps after obtaining a second conversion table in the iteration step.
[Fig. 3A] Fig. 3 shows an example for explaining a clustering method when using new references. Fig. 3A is a diagram showing an example of obtaining ID groups using a second reference.
[Fig. 3B] Fig. 3B shows an example of obtaining a second recombined ID group using the obtained ID groups.

### DESCRIPTION OF EMBODIMENTS

Hereinafter, modes for carrying out the present invention will be described using drawings. The present invention is not limited to the modes described below and includes those appropriately modified from the following modes within the scope obvious to those skilled in the art.

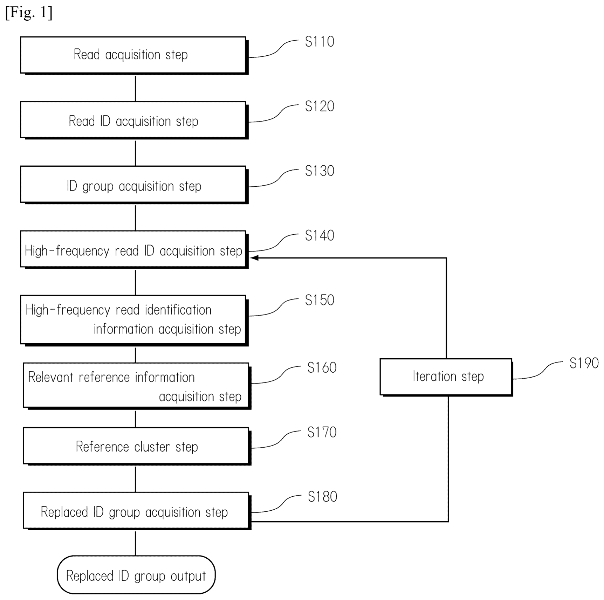

Fig. 1 is a flowchart showing an example of steps of a clustering method. As shown in Fig. 1, the sequence clustering method includes a read acquisition step (S110), read ID acquisition step (S120), ID group acquisition step (S130), high-frequency read ID acquisition step (S140), high-frequency read identification information acquisition step (S150), relevant reference information acquisition step (S160), reference cluster step (S170), and replaced ID group acquisition step (S180). As shown in Fig. 1, this method may further include an iteration step (S190). This method is basically implemented by computers or servers. The sequence clustering method means consolidating references into groups (clusters). By performing clustering, the number of references to be examined can be reduced, and analysis of specimen-derived reads can be performed rapidly.

The computer has an input unit, output unit, control unit, calculation unit, and storage unit, and each element is connected by buses and the like so that information can be exchanged. For example, control programs may be stored in the storage unit, or various information may be stored. When predetermined information is input from the input unit, the control unit reads out the control program stored in the storage unit. The control unit appropriately reads out information stored in the storage unit and transmits it to the calculation unit. The control unit also appropriately transmits input information to the calculation unit. The calculation unit performs calculation processing using various received information and stores it in the storage unit. The control unit reads out calculation results stored in the storage unit and outputs them from the output unit. In this way, various processes and steps are executed. Each unit and each means executes these various processes. The computer may have a processor, and the processor may realize various functions and steps. The computer may be standalone. The computer may have some functions distributed between a server and a terminal. In that case, it is preferable that the server and terminal can exchange information through networks such as the Internet or intranet. The computer may include a processor and memory coupled to the processor. The memory may store commands, and when the commands are executed by the processor, they may cause the computer to perform various steps or function as various elements. The computer may construct learning models by providing various teacher data and realize various calculations through machine learning. In this case, the computer may execute various analyses using learning models created through AI (artificial intelligence) machine learning and deep learning.

The sequence clustering method may be implemented by a sequence clustering device. This device is implemented by a computer and includes read acquisition means for performing the read acquisition step (S110), read ID assignment means for performing the read ID acquisition step (S120), ID group acquisition means for performing the ID group acquisition step (S130), high-frequency read ID acquisition means for performing the high-frequency read ID acquisition step (S140), high-frequency read-containing ID group acquisition means for performing the high-frequency read identification information acquisition step (S150), relevant reference information acquisition means for performing the relevant reference information acquisition step (S160), reference cluster means for performing the reference cluster step (S170), and replaced ID group acquisition means for performing the replaced ID group acquisition step (S180). This device may further include iteration means for performing the iteration step (S190).

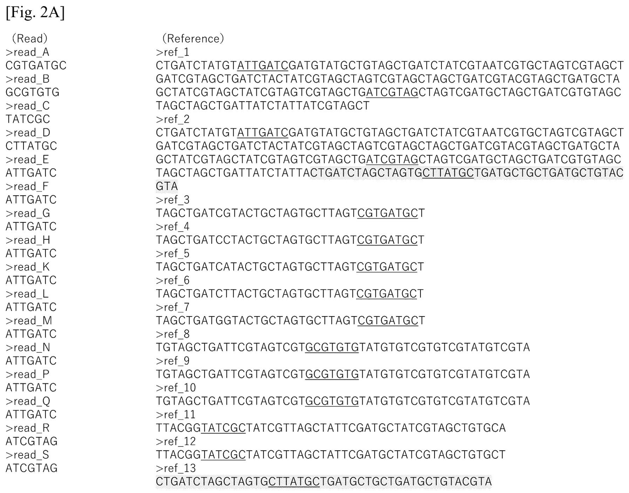

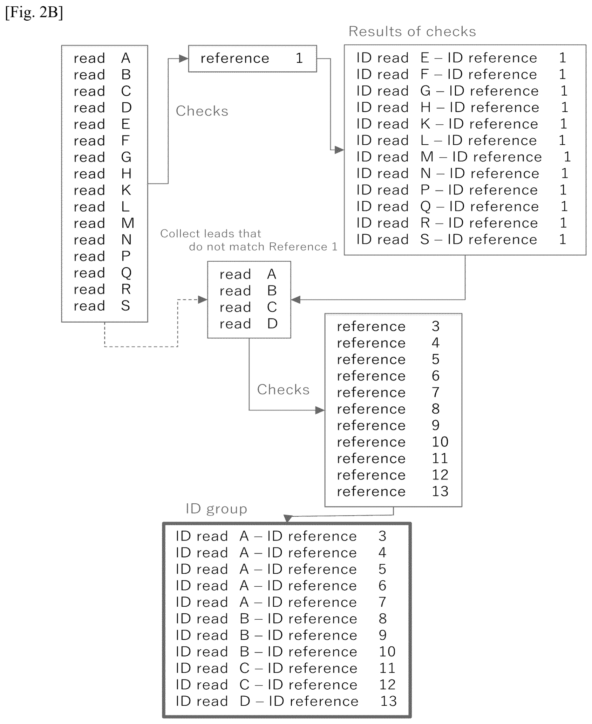

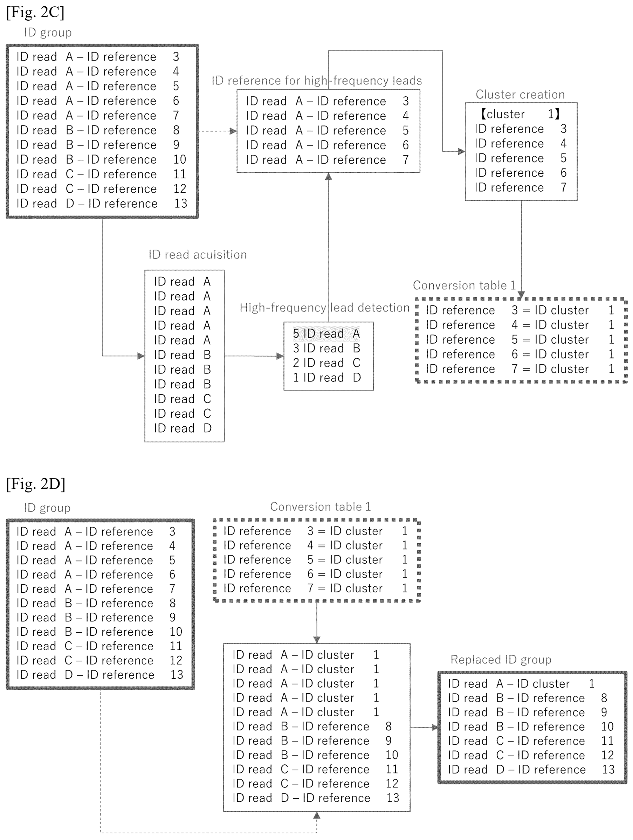

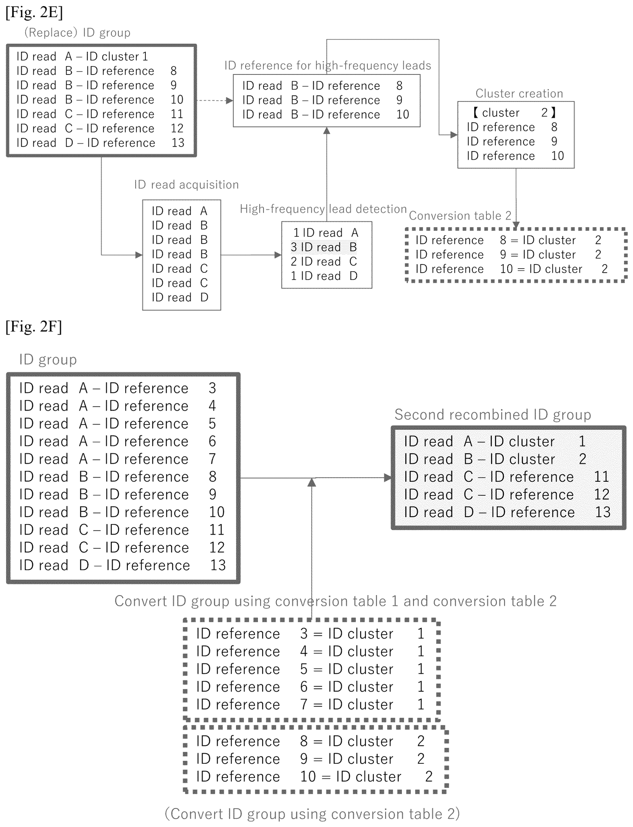

Fig. 2 shows an example for explaining the clustering method of this invention. Fig. 2A shows examples of read and reference sequences and identification information. In the example of Fig. 2A, for simplicity, specimen-derived reads A-S and references 1-13 are handled. Reference 1 is a host-derived sequence (host cell genome sequence). Reference 2 is a sequence derived from a host infected with a virus. References 3-13 are either recorded in a virus-related database or stored in the database as virus references related to specimens. In this example, reads A-D are reads derived from other than the host. Reads E-S are reads derived from reference 1 or 2. Host cell-derived nucleic acids are also included in specimens and are read together with reads derived from other than the host by sequencers and the like. Therefore, host-derived reads are also included as specimen-derived reads. In the example of Fig. 2A, the host cell that became the source of reference 2 was infected with a virus (reference 13), so it has the sequence of reference 13 as a partial sequence. References 3-7 relate to the same virus with different specimen acquisition dates. References 8-10 also relate to the same virus with different specimen acquisition dates. References 11-13 are sequences derived from viruses and the like.

What is shown in Fig. 2A is exemplary, and in actual clustering performed, the length of read base sequences was about 50 to 200 bases long. The length of references was about 2.6 billion bases long for references 1 and 2, which are host cell genomes, and references 3-13 were about hundreds to tens of thousands of bases long. The base length of SARS-CoV-2 was about 20,000 bases long. The base length of reads may be 5 bases or more and 100,000 bases or less, 10 bases or more and 10,000 bases or less, or 10 bases or more and 1,000 bases or less. The base length of references is preferably longer than the base length of reads, preferably 10,000 bases or more and 50 billion bases or less, and may be 100,000 bases or more and 10 billion bases or less, or 1 million bases or more and 10 billion bases or less.

The read acquisition step (S110) is a step of acquiring reads that are base sequences derived from specimens. An example of a specimen is a bio-pharmaceutical. For example, the clustering device is connected to a sequencer. The sequencer analyzes base sequences contained in specimens. The analyzed base sequences (reads) are input to the clustering device. In this way, the clustering device acquires reads. The clustering device may acquire reads by dragging and dropping read base sequences into software in the clustering device. The acquired read base sequences are appropriately stored in the storage unit. Hereinafter, information obtained by the clustering device in this way may be appropriately stored in the storage unit. This step may acquire reads (and read IDs) by, for example, inputting base sequences derived from specimens analyzed by the sequencer into the clustering device.

The read ID acquisition step (S120) is a step of acquiring identification information on the reads. For example, the clustering device may acquire identification information on each read stored together with reads when acquiring reads. The clustering device may also assign identification information to each read stored in the storage unit and store it in the storage unit. In this way, the clustering device acquires read identification information.

In the example of Fig. 2A, since there were 18 reads, reads A-S were input to the clustering device together with their sequence information and stored in the storage unit of the clustering device. Read base sequences may be input, and identification information (reads A-S) may be assigned to each input read base sequence and stored in the storage unit.

The ID group acquisition step (S130) checks, with reference to a database including a plurality of base sequences of references and storing the base sequence of each reference in association with an organism species and identification information on each reference, the reads against the references included in the database. This step acquires an ID group that is a group of the read identification information and the reference identification information in a case where the base sequences of the references include the base sequences of the reads. Examples of base sequences of references included in the database are base sequences derived from viruses, bacteria, and molds (viruses, etc.) that may be included in bio-pharmaceuticals. The above database may also store references other than those derived from viruses, etc. The checking process is similar to the process described earlier.

A preferred example of the ID group acquisition step (S130) includes a first read group acquisition step (S131) and a first ID group acquisition step (S132). It is preferable that the clustering device has first read group acquisition means for performing the first read group acquisition step (S131) and first ID group acquisition means for performing the first ID group acquisition step (S132).

The first read group acquisition step (S131) is a step of checking the reads against a first host-derived reference that is a reference derived from a host of the specimen, and obtaining a first read group of reads other than reads whose base sequences are included in the first host-derived reference sequence. Host-derived references of specimens may be obtained from the database or from storage units other than the database. The host of the specimen means the host used to obtain various components used in the specimen. For example, in the case of pharmaceuticals for humans, human cells may be used when obtaining pharmaceuticals, and in that case, humans may be the host. When a certain component included in the specimen is cultured using fetal bovine serum (FBS), an example of the specimen host is cattle. The first ID group acquisition step (S132) is a step of checking the first read group against the references included in the database, and acquiring a first ID group.

In the example of Fig. 2A, reference 1, which is a reference of host-derived sequences (host cell genome sequences), corresponds to the first host-derived reference sequence.

Fig. 2B is a diagram for explaining an ID group acquisition step including a first read group acquisition step and a first ID group acquisition step. Reference 1 may be obtained from the database or from storage devices other than the database. As shown in Fig. 2B, the clustering device checks reads A-S against reference 1. Specifically, the clustering device reads out the base sequence of read A from the storage unit and performs checking processing to determine whether it is included in the base sequence of reference 1. The clustering device also performs this work for reads B and subsequent reads. In the example of Fig. 2, the base sequences of reads E-S were included in the base sequence of reference 1. Therefore, the clustering device removes reads E-S from reads A-S and stores reads A-D as the first read group in the storage unit.

In the example of Fig. 2B, ID groups (first ID groups) are acquired using the first read group as reads. In the example of Fig. 2B, the database stores references 3-13 as virus-related references. This database stores information about organism species and base sequence information for each of the reference identification information of references 3-13.

The clustering device checks each of reads A-D included in the first read group against references 3-13. Specifically, the clustering device reads out the base sequence of read A from the storage unit and performs checking processing to determine whether the base sequence of read A is included in the base sequences of references 3-13. This work is also performed for reads B-D. In the example of Fig. 2, examples of ID groups include multiple ID groups such as ID read A - ID reference 3. However, if, for example, a partial sequence of the reference indicated by ID reference 3 matches the sequence of the read indicated by ID read B, a group called ID read B - ID reference 3 may exist. In this way, ID groups are obtained and stored in the storage unit.

A preferred example of the ID group acquisition step (S130) further includes a deduplication step (S135). The deduplication step (S135) is a step of leaving, in a case where there are a plurality of ID groups with an identical pair of the read identification information and the reference identification information, only one of the ID groups and removing the other ID groups. For example, data of combinations of IDs of specimen-derived reads and IDs of database-derived sequences may be arranged in a single column as rows. Among the data, for completely matching rows, one row should be left and the rest should be removed. For example, when two or more ID read A - ID reference 3 exist (when the sequence of read A exists in two or more places in the sequence of reference 3), only one ID group should be left and other ID groups (groups with the same read and reference IDs) should be removed. In this way, deduplicated ID groups can be obtained. When there are no duplicates in ID groups, the ID groups obtained in the previous step become the deduplicated ID groups. Therefore, in subsequent steps, deduplicated ID groups are also simply expressed as ID groups. The clustering device should read out read identification information and reference identification information included in each ID group, and when there are multiple ID groups where these match, leave one ID group and remove others.

In the example of Fig. 2B, there are no multiple ID groups with the same pair of read identification information and reference identification information included in ID groups. Therefore, there is no need to perform the deduplication step (S135). Although not in the example of Fig. 2B, for example, when there are multiple ID groups of ID read A - ID reference 3, any one ID group should be left and other ID groups should be removed.

The high-frequency read ID acquisition step (S140) is a step of obtaining the number of pieces of read identification information included in ID groups and obtaining high-frequency read identification information that is identification information on high-frequency reads that are reads with high appearance rates. The clustering device reads out ID groups stored in the storage unit, performs calculations to obtain the number of read identification information included in ID groups, and obtains the number of identification information for each read included in ID groups. The clustering device then compares the obtained numbers of identification information for each read. In this way, the clustering device can obtain high-frequency read identification information.

Fig. 2C is a diagram for explaining steps after acquiring ID groups. In the example of Fig. 2C, ID groups include five ID read As, and ID read A is high-frequency read identification information.

The high-frequency read identification information acquisition step (S150) is a step of obtaining high-frequency reference identification information that is the reference identification information grouped with the high-frequency read identification information included in the ID groups. The clustering device reads out high-frequency read identification information from the storage unit and reads out reference identification information grouped with high-frequency read identification information in ID groups. In this way, the clustering device can obtain high-frequency reference identification information.

In the example of Fig. 2C, among ID groups, there are five that include ID read A, so the reference identification information (references 3-7) in those five ID groups is high-frequency read identification information. In the example of Fig. 2C, high-frequency read identification information is collected to form a cluster (cluster 1).

The relevant reference information acquisition step (S160) is a step of acquiring, using the high-frequency reference identification information, information including an organism species of the high-frequency reference sequences stored in association with the high-frequency reference identification information from the database. The clustering device uses high-frequency reference identification information to acquire information including organism species of high-frequency reference sequences stored in association with high-frequency reference identification information from the database. Examples of information including organism species of high-frequency reference sequences may include organism species (e.g., virus names) as well as one or more of information about reference acquisition dates, information about reference sequences, and information about past clusters. The clustering device may identify organism species of high-frequency reads from obtained information about organism species. The clustering device also aggregates information about organism species of references related to high-frequency reads, so displaying these makes it easier to judge organism species of high-frequency reads. By performing the relevant reference information acquisition step (S160), judgment of whether to group as clusters can be made not only from identification information (IDs) but also using organism species names such as viruses. In the relevant reference information acquisition step (S160), when there is one type of organism species for high-frequency reads, it may be judged that high-frequency reads are derived from that organism species. When there are multiple types of organism species for high-frequency reads, the high-frequency organism species may be taken as the organism species of high-frequency reads. Then, clustering below may be performed only for high-frequency reads that are that high-frequency organism species. Furthermore, when organism species of high-frequency reads include those derived from specimens, clustering below may be performed excluding those high-frequency reads.

The relevant reference information acquisition step (S160) is an optional step and may be omitted. As described above, references related to one of the organism species obtained by the relevant reference information acquisition step (S160) may be used for clustering described later.

In the example of Fig. 2C, information such as organism species of references 3-7 is read out in relation to ID read A. The clustering device may judge that ID read A is derived from coronavirus based on, for example, the read organism species. For example, when references 3-7 are references related to coronavirus, it may be judged that there is a possibility that coronavirus was mixed in the specimen bio-pharmaceutical. Even when references 3-6 are references related to coronavirus and reference 7 is a reference derived from mold or host, it may be judged that there is a possibility that coronavirus was mixed in the specimen bio-pharmaceutical. In this case, references 3-6 may be clustered in the reference cluster step described later.

The reference cluster step (S170) is a step of allowing the high-frequency reference identification information to be replaced with first cluster identification information that is a new piece of identification information. In this step, information for replacing high-frequency reference identification information with first cluster identification information that is a new piece of identification information should be stored in the storage unit. An example of such information is something that stores high-frequency reference identification information in association with first cluster identification information (e.g., a table). The clustering device stores first cluster identification information in the storage unit in association with, for example, high-frequency reference identification information. Then, the clustering device becomes able to convert high-frequency reference identification information into first cluster identification information.

In the example of Fig. 2C, ID references 3-7 are made replaceable with ID cluster 1. In the example of Fig. 2C, a first conversion table (conversion table 1) was obtained. Using this table, ID references 3-7 can be rewritten to unified IDs like ID cluster 1.

The replaced ID group acquisition step (S180) is a step of acquiring a replaced ID group by converting the high-frequency reference identification information included in the ID groups into the first cluster identification information. Here, an example of ID groups is ID groups obtained by the ID group acquisition step (S130). The clustering device stores read identification information and reference identification information of each ID group. Therefore, when reference identification information of each ID group is high-frequency reference identification information, the clustering device should convert high-frequency reference identification information into cluster identification information and acquire replaced ID groups. When converting high-frequency reference identification information into cluster identification information results in multiple ID groups with the same pair of read identification information and reference identification information, the clustering device may leave only one ID group among them and remove other ID groups. In this way, the clustering device can acquire replaced ID groups.

Fig. 2D is a diagram for explaining an example of clustering processing after obtaining a conversion table. In the example of Fig. 2D, ID references 3-7 are replaced with ID cluster 1. Then, five groups of ID read A - ID cluster 1 are generated. Therefore, the clustering device may leave one ID group of ID read A - ID cluster 1 and remove the remaining four. In this way, replaced ID groups can be obtained. In this way, relationships between references can be measured using reads. As shown in Fig. 2D, the clustering device can acquire replaced ID groups.

A preferred example of the sequence clustering method may perform an iteration step (S190) that performs the high-frequency read ID acquisition step, high-frequency read identification information acquisition step, relevant reference information acquisition step, and replaced ID group acquisition step using replaced ID groups in a case where replaced ID groups include a plurality of pieces of identification information on a certain read. The clustering device obtains the number of each read included in ID groups in the high-frequency read ID acquisition step (S140) and stores it in the storage unit. The clustering device should judge whether to perform the iteration step (S190) for the read with the next highest number after high-frequency reads as a new high-frequency read. For example, the clustering device should store thresholds in the storage unit and perform the iteration step (S190) when the number included in ID groups of the read with the next highest number after high-frequency reads is equal to or greater than the threshold or greater than the threshold. In this way, in this invention, clustering of references can be performed by measuring similarity between references using reads.

Fig. 2E is a diagram for explaining an example of steps up to obtaining a second conversion table in the iteration step. In the example of Fig. 2E, processing similar to ID read A described earlier is performed for ID read B. Then, a second cluster (cluster 2) is obtained and a second conversion table (conversion table 2) is obtained.

Fig. 2F is a diagram for explaining an example of steps after obtaining a second conversion table in the iteration step. As shown in Fig. 2F, in this example, a second recombined ID group with advanced clustering was obtained. The example of Fig. 2F shows performing the iteration step once. This iteration step may be performed multiple times. For example, when the iteration step is performed further, references 11 and 12 become cluster 3.

A preferred example of the sequence clustering method includes a second read group acquisition step (S133) and a second ID group acquisition step (S134). Then, the various steps described above are performed using the newly acquired second ID groups. In this way, by comparing first ID groups and second ID groups, or comparing first replaced ID groups and second replaced ID groups, cases where the host itself was infected with a virus can be judged. In other words, false negatives can be prevented. This is because when checking is performed between host-derived references and reads, if the host itself was infected with a virus, even if that virus is included in the specimen, there is a risk that it would be judged as host-derived and negative (not containing virus).

Using replaced ID groups (nth replaced ID groups) acquired as described above, whether specimens are contaminated with viruses, etc. can be checked using fewer ID groups. Therefore, this specification also provides a method for checking contamination status of specimens using the clustering method described above. That is, when virus-derived references exist among replaced ID groups, the computer should judge that the specimen is contaminated with viruses, etc.

The second read group acquisition step (S133) is a step of checking the reads against a second host-derived reference that is a reference derived from the host of the specimen, and obtaining a second read group of reads other than reads whose base sequences are included in the second host-derived reference sequence. The second host-derived reference sequence and the first host-derived reference sequence are different sequences.

The second ID group acquisition step (S134) is a step of checking the second read group against the references included in the database, and acquiring a second ID group.

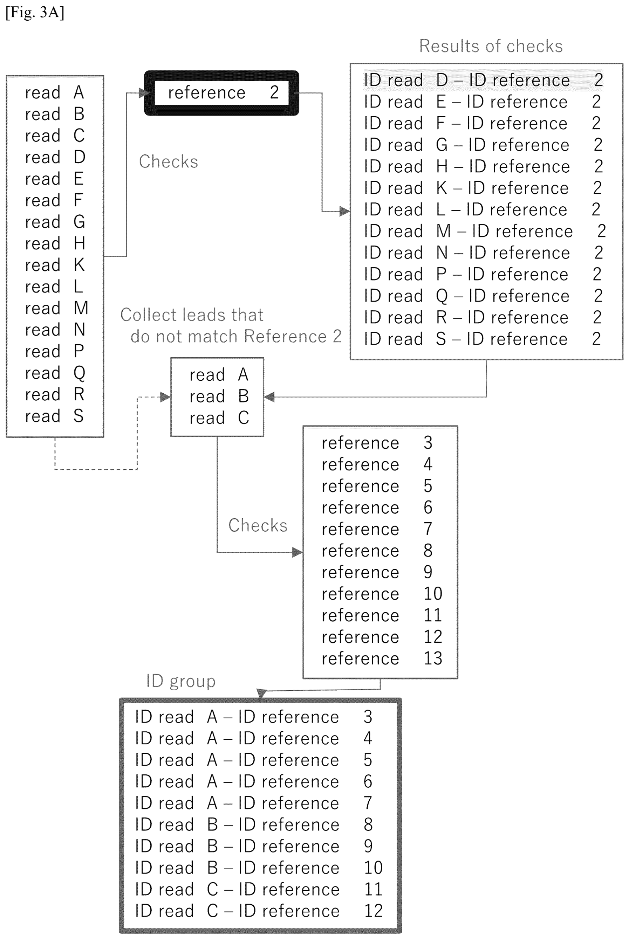

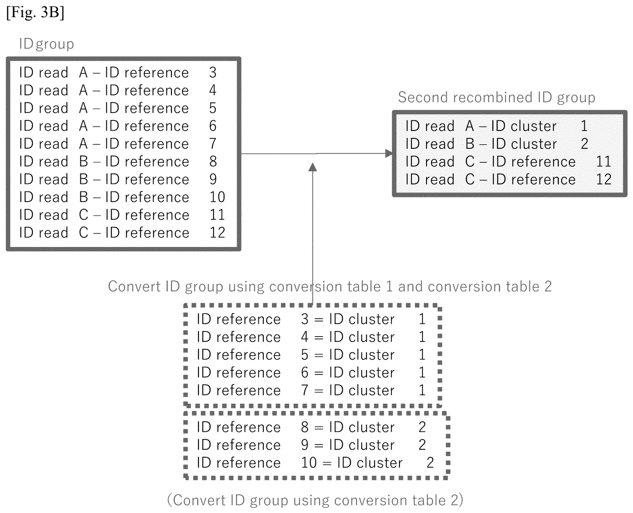

Fig. 3 shows an example for explaining a clustering method when using new references. Fig. 3A is a diagram showing an example of obtaining ID groups using a second reference. Fig. 3B shows an example of obtaining a second recombined ID group using the obtained ID groups. In the cell that became the source of reference 2, it was infected with the virus of reference 13 before acquiring the reference genome. Therefore, the virus sequence of reference 13 was mixed into reference 2 as a host cell-derived sequence. The virus of reference 13 being included in the specimen would be overlooked. When removing host-derived sequences from reads through checking, false negatives can be suppressed by using multiple independently acquired reference sequences.

A computer for performing sequence clustering comprises a processor and a memory coupled to the processor. The memory stores a program including a command. When executed by the processor, the command causes the computer to execute a sequence clustering method including the read acquisition step (S110), read ID acquisition step (S120), ID group acquisition step (S130), high-frequency read ID acquisition step (S140), high-frequency read identification information acquisition step (S150), relevant reference information acquisition step (S160), reference cluster step (S170), and replaced ID group acquisition step (S180). When executed by the processor, the command may further cause the computer to execute the iteration step (S190).

The above program is a program for causing a computer to execute a sequence clustering method including the read acquisition step (S110), read ID acquisition step (S120), ID group acquisition step (S130), high-frequency read ID acquisition step (S140), high-frequency read identification information acquisition step (S150), relevant reference information acquisition step (S160), reference cluster step (S170), and replaced ID group acquisition step (S180). The program may further cause the computer to execute the iteration step (S190).

A non-transitory information recording medium stores the above program. Examples of recording media are hard disks, CDs, CD-ROMs, floppy disks, Blu-ray discs, USB memory, and SD cards.

### INDUSTRIAL APPLICABILITY

This invention can be utilized in the pharmaceutical industry and reagent industry.

## Claims

1. A sequence clustering method comprising:
a read acquisition step of acquiring a read that is a base sequence derived from a specimen; a read ID acquisition step of acquiring identification information on the read;
an ID group acquisition step of checking, with reference to a database including a plurality of base sequences of references and storing the base sequence of each reference in association with an organism species and identification information on each reference, the read against the references included in the database, and acquiring an ID group that is a group of the read identification information and the reference identification information in a case where the base sequences of the references include the base sequence of the read;
a high-frequency read ID acquisition step of obtaining the number of pieces of the read identification information included in the ID group, and obtaining high-frequency read identification information that is identification information on a high-frequency read that is the read with a high appearance ratio;
a high-frequency read identification information acquisition step of obtaining high-frequency reference identification information that is the reference identification information grouped with the high-frequency read identification information included in the ID group;
a relevant reference information acquisition step of acquiring, using the high-frequency reference identification information, information including an organism species of the high-frequency reference sequence stored in association with the high-frequency reference identification information from the database;
a reference cluster step of allowing the high-frequency reference identification information to be replaced with first cluster identification information that is a new piece of identification information;
and a replaced ID group acquisition step of acquiring a replaced ID group by converting the high-frequency reference identification information included in the ID group into the first cluster identification information.

2. The method according to claim 1, wherein the ID group acquisition step includes:
a first read group acquisition step of checking the read against a first host-derived reference that is a reference derived from a host of the specimen, and obtaining a first read group of reads other than a read whose base sequence is included in a first host-derived reference sequence; and a first ID group acquisition step of checking the first read group against the references included in the database, and acquiring a first ID group.

3. The method according to claim 2, wherein the ID group acquisition step further includes:
a second read group acquisition step of checking the read against a second host-derived reference that is a reference derived from the host of the specimen, and obtaining a second read group of reads other than a read whose base sequence is included in a second host-derived reference sequence, the second host-derived reference sequence and the first host-derived reference sequence being different sequences; and
a second ID group acquisition step of checking the second read group against the references included in the database, and acquiring a second ID group.

4. The method according to claim 2 or 3, wherein the specimen is a bio-pharmaceutical, and the base sequences of the references included in the database are base sequences derived from a virus with a possibility of being included in the bio-pharmaceutical.

5. The method according to claim 4, wherein the ID group acquisition step further includes a deduplication step of leaving, in a case where there are a plurality of ID groups with an identical pair of the read identification information and the reference identification information, only one of the ID groups and removing the other ID groups.

6. The method according to claim 4, wherein in a case where the replaced ID group includes a plurality of pieces of identification information on a certain read,
the high-frequency read ID acquisition step,
the high-frequency read identification information acquisition step,
the relevant reference information acquisition step, and
the replaced ID group acquisition step are performed using the replaced ID group.

7. A computer for performing sequence clustering, comprising:
a processor; and a memory coupled to the processor, wherein the memory stores a program including a command, and when executed by the processor, the command causes the computer to execute the sequence clustering method according to claim 1.

8. A program for causing a computer to execute the sequence clustering method according to claim 1.

9. A non-transitory information recording medium storing the program according to claim 8.
